# EUROPEAN PATENT SPECIFICATION

(11) **EP 1 024 361 B1**
(45) Date of publication and mention of the grant of the patent: **09.04.2003**
(21) Application number: 00200276.4
(22) Date of filing: 26.01.2000
(51) Int. Cl.: G01N 33/32

(54) **Method and apparatus for determining the behavior of a film layer of printing ink**
Verfahren und Vorrichtung zur Feststellung des Verhaltens einer Drucktintefilmschicht
Méthode et appareil pour la détermination du comportement d'une couche mince d'encre

(30) Priority: 26.01.1999 NL 1011135
(43) Date of publication of application: 02.08.2000
(73) Proprietor: Mechatron Holdings B.V., 8445 PE Heerenveen (NL)
(72) Inventor: de Jong, Guido, 9084 CH Goutum (NL); Massolt, Peter Robert, 8446 JP Heerenveen (NL); Massolt, Frits, 9203 RZ Drachten (NL); Huls, Frank, 7071 VC Ulft (NL)
(74) Representative: Prins, Adrianus Willem

(56) References cited:
- EP-A- 0 355 571
- EP-A- 0 741 295
- DE-A- 3 410 393
- DE-A- 3 711 864
- US-A- 5 341 734
- PATENT ABSTRACTS OF JAPAN vol. 014, no. 277 (M-0985), 15 June 1990 (1990-06-15) & JP 02 084338 A (MITSUBISHI HEAVY IND LTD), 26 March 1990 (1990-03-26)

## Description

The invention relates to a method for determining the behavior of a film layer of printing ink, according to the introductory part of claim 1. The invention also relates to an apparatus according to the introductory part of claim 16, designed for performing such method.

Such method and such apparatus are known A from applicant's German patent application DE 34 10 393A.

When such apparatus is used, the changing of the content of the component in question is effected by taking up the component whose content is to be changed (in this case water) with a water applying roller to which water is applied and delivering it therewith to the film layer of printing ink applied to a circulating surface in the form of a revolving surface of a central roller.

The object of the invention is to provide that during the changing of the content of the component or the components, the changes are made in a more homogeneous manner so as to obtain more representative results.

According to the present invention, this object is realized by performing a method of the type initially indicated in accordance with the characterizing part of claim 1. For performing the method according to the invention, the invention also provides an apparatus according to claim 16.

Due to the fact that the film layer is formed from an ink supply in a container and in contact with the revolving film layer-carrying surface and the content of the component or the components in that ink supply in the container is changed, more representative results are obtained than in the case where the content of the component is changed through addition to or removal via an outwardly facing, free surface of the film-shaped ink layer. Moreover, each time when it passes through the ink supply in the container, the ink film is at least partially broken down and formed afresh, so that a constant and even structure of the ink film can also be maintained during longer tests.

Particular embodiments of the invention are laid down in the dependent claims.

Further objects, structural aspects, effects and details of the invention will appear from the claims and the following description, in which reference is made to the accompanying drawings. In these drawings:

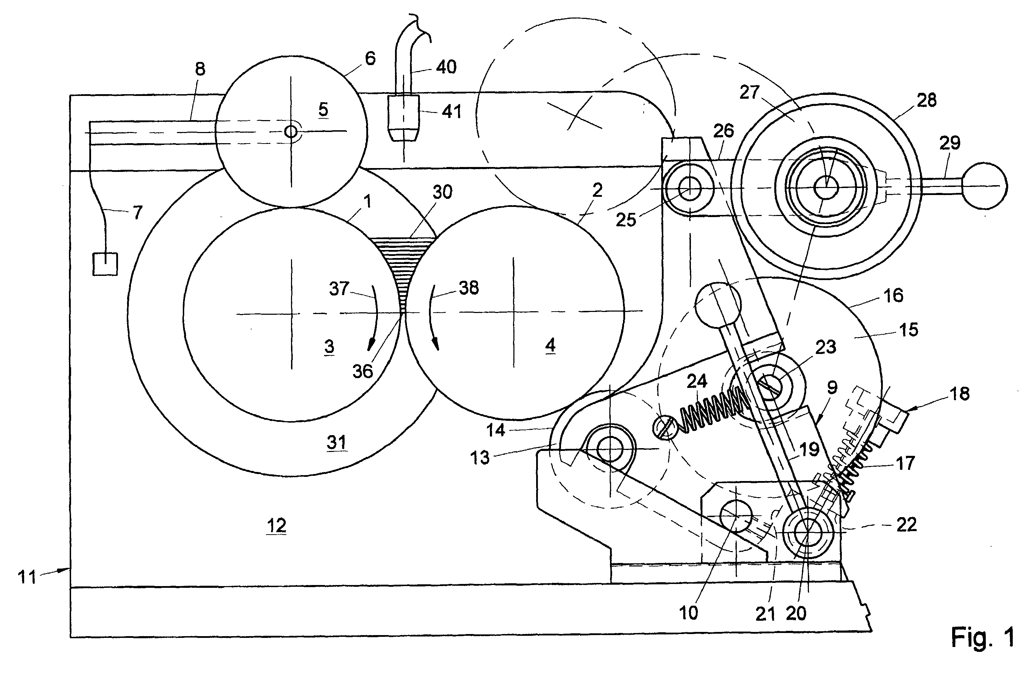

Fig. 1 is a sectional side elevation of an example of an apparatus according to the invention taken on the line I-I in Fig. 2;
Fig. 2 is a front view of the apparatus according to Fig. 1, viewed from the right-hand side in Fig. 1; and
Fig. 3 is a more schematic, partially cutaway top plan view of a roller assembly of the apparatus according to Figs. 1 and 2.

The apparatus which, by way of example, is shown in the drawings and described hereinbelow and the method to be performed therewith, also described by way of example, presently constitute the most preferred embodiments of the invention.

The apparatus proposed serves for establishing the behavior of a printing ink at different contents of at least one component, such as a solvent, water, an aqueous substance (for instance with alcohol), a pigment or another ink component. In the present example, the above is described with reference to tests for determining the behavior of an offset printing ink upon variation of the water content, which forms one of the major utilizations of the proposed apparatus and the proposed method. The capacity of absorbing and expelling water is of particular importance for the processing properties of a printing ink. In practice, when the structure of the film layer is homogeneous directly during the formation thereof, its behavior proves to be considerably more representative of the behavior of the ink than when the content of water in printing ink divided into a film is changed directly.

The apparatus has two circulatable surfaces for carrying a film layer of printing ink in the form of metal (steel) circumferential surfaces 1, 2 of cylinders 3, 4, suspended for rotation about center lines thereof from vertical flanges 12 of a main frame 11 of the apparatus.

For performing tests on the film layer, two sets of instruments are provided.

Firstly, there is provided a set of instruments for measuring the tack of an ink layer. According to this example, this set of instruments consists of, inter alia, a measuring roller 5 whose revolving surface 6, in operative condition, unrolls over the circumferential surface 1 of the roller 3, or at least over an ink layer formed on that circumferential surface 1, and leaf spring-shaped power sensors which are known per se and hence only shown schematically and which are connected, via arms 8, to the measuring roller 5. The measuring roller 5 is suspended from the arms 8 for rotation about its axis.

Secondly, there is provided a set of instruments for causing to rotate along therewith a printing plate roller carrying a printing plate having a partially hydrophilic and a partially hydrophobic surface, and a trial printing roller which, in turn, rotates along with the printing plate roller.

For this purpose, there is provided a pivotable roller support 9, pivotable about an axis 10 relative to the main frame 11 of the apparatus. Rotatably suspended in the pivotable roller support 9 are an ink transfer roller 13 whose circumferential surface 14 is manufactured from rubber and a printing plate roller 15 whose outer surface is formed by a printing plate 16 having a partially hydrophilic and partially hydrophobic surface. A spring 17 of a settable tensioner 18 engages, in operative condition, the pivotable roller support 9 for pressing it down on the side of the spring 17, whereby it is forced upwards on the side of the ink transfer roller 13 and the ink transfer roller 13 is pressed against one of the cylinders 4. There is further provided a lever 19 pivotable about an axis 20 relative to the frame 11 of the apparatus. The lever 19 is coupled to a projection 21 which, in turn, is located opposite a shoulder 22 of the roller support 9. By operating the lever 19 such that the projection 21 forces the roller support 9 upwards at the location of the shoulder 22, the roller support 9 is pivoted against a force exerted by the spring 17 and the ink transfer roller 13 comes loose from the cylinder 4. The printing plate roller 15 is suspended for displacement relative to the ink transfer roller 13 such that the distance between the axes of the printing plate roller 15 and the ink transfer roller 13 is variable. Between axis stubs 23 of the printing plate roller 15 and the roller support 9, springs 24 are tensioned, urging the printing plate roller 15 against the ink transfer roller 13 at a constant pressure.

By causing the ink transfer roller 13 and the printing plate roller 15 to rotate along with the cylinder 4, the clearance of the hydrophilic surface at different water contents can for instance be observed. Thus, for instance, the sensitivity of the ink to variations in the water content can be tested.

Further, the roller support 9 supports a single arm 26, pivotable about an axis 25 and supporting a rotatably suspended trial printing roller 27 having an outer surface 28. By pivoting the arm 26 down, the outer surface 28 of the trial printing roller 27 can be pressed against the outer surface 16 of the printing plate roller 15, so that an ink pattern formed on the printing plate roller 15 is taken over on the trial printing roller. Extending in line with the arm 26 is an operating arm 29 whereby the trial printing roller 27 can be operated. An axis stub of the trial printing roller 27 is mounted on the arm 26 by a knurled nut knob 42. By loosening the knurled nut knob 42, the trial printing roller 27 can readily be detached from the arm 26. By subsequently mounting the trial printing roller 27 in a trial printing machine, a proof can be made on a substrate such as paper, for examining the sharpness and density of a print of a pattern or area taken from the printing plate roller 15. Due to the one-sided attachment of the trial printing roller 27 to the single arm 26, it can be mounted and demounted in a simple manner.

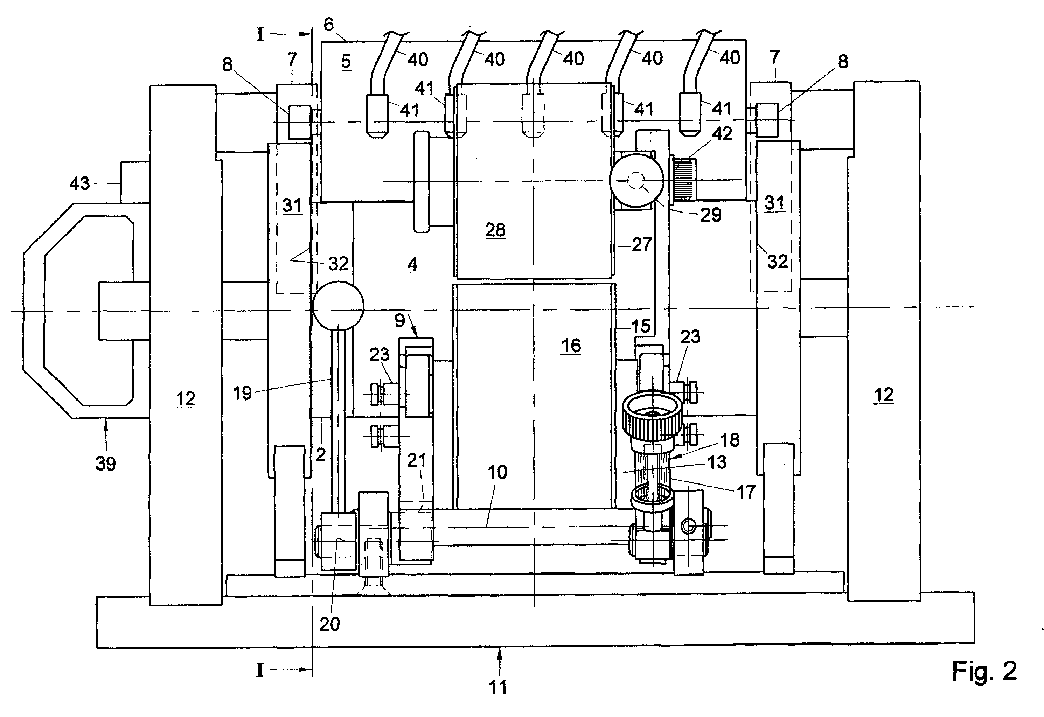

By means of the arm 29, the trial printing roller 27 is also pivotable about the point of rotation 25 into a position against the circumferential surface 2 of the cylinder 4, as shown in dash-dot lines in Fig. 2. This makes it possible to take up ink for making a proof directly from the circumferential surface 2 of the cylinder 4 and, accordingly, to make solid proofs without the interposition of a printing plate on the roller 15. Proofs where the sharpness of the image is not concerned can thus be carried out faster and more efficiently.

The cylinders 3 and 4 form in mutual cooperation a container for taking up an ink supply 30 in contact with portions of the circulatable surfaces 1, 2, while the circulatable surfaces 1, 2, moving parallel to the parts of those faces in the container, are movable through the container. For keeping the ink supply 30 between the cylinders 3, 4, the cylinder 3 is provided with flanges 31 which sealingly abut against end faces 32 of the cylinder 4.

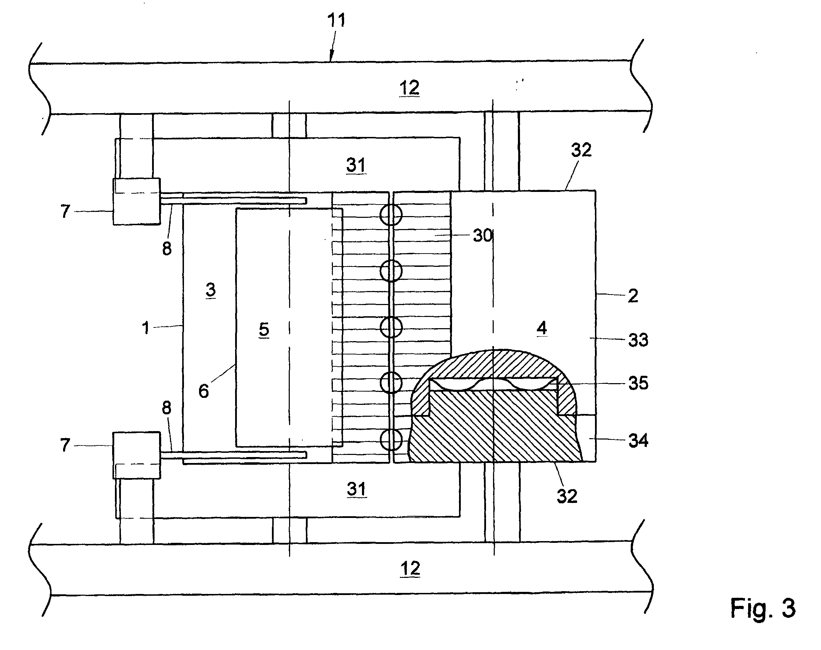

To guarantee an adequate seal between the flanges 31 and the end surfaces 32, sliding along each other in operation, the cylinder 4 is of sectional design and axially expandable over a very slight distance by a spring element 35 located between the parts 33, 34 (see Fig. 3). Thus, the end surfaces 32 abut with some bias against facing surface parts of the flanges 31.

Between the circulatable surfaces 1, 2 of the rollers 3, 4 suspended side by side, a gap 36 is present of preferably 5-1000 µm, and for use with inks of usual viscosity, preferably 10-80 µm and more in particular 20-40 µm, so that upon rotation of the rollers 3, 4 in the sense of rotation indicated by the arrows 37, 38, a film of the desired thickness is formed in the area of the gap 36 and the ink supply 30 above the gap 36 is retained. Although the width of the gap 36 may be flexible, it is preferably substantially rigidly maintained at the desired width, to enable accurate control of the thickness of the film layer.

For driving the rollers 3, 4, the roller 4 is coupled to a drive motor 39. The roller 3 is moved along by the driven roller 4.

In use, an ink supply 30 is provided above the gap 36 between the rollers 3, 4 and upon rotation of the rollers 3, 4, film layers of the printing ink are formed on the circulating outer surfaces 1, 2 of the cylinders 3, 4. With the above-described sets of instruments, tests are subsequently performed on the film layers to determine the characteristics of the printing ink. By changing the water content of the ink from which the film layer is formed and performing tests on the film layer having the changed water content, the effect of the different water contents on the tack and the printing properties of the ink can be established very fast. Since the film layer is formed from the ink supply 30 in contact with the revolving surface 1, 2 and the water content in the ink supply is changed, a homogeneous film structure is promoted and an ink behavior is realized which is highly representative of the behavior of ink in practice. Moreover, each time when the revolving surface 1, 2 on which the ink film is located moves through the ink supply, the ink film is at least partially broken down and formed afresh, so that also when the apparatus is run for a longer period without interruption, a uniform and controlled composition of the ink layer is maintained.

Although the changing of the water content can, for instance, be effected through evaporation of water from the ink (both from ink in film form and from ink in the supply 30) while the apparatus is in operation, or through the addition of water to the ink supply 30 by means of a pipette or the like, for an efficient, user-friendly and controlled operation of the apparatus it is advantageous that it is provided with a discharge structure having feed lines 40 and nozzles 41 for the dosed feed of water and, if so desired, other components to the container for retaining an ink supply 30.

Through the addition of water to the ink supply 30, or of another component whose content is to be varied, it is achieved that the added component is first distributed over the ink supply and the ink film is then formed directly from ink having the increased content of the component in question.

In fact, adding a component in an accurately dosed manner can be effected in the simplest manner when said component is liquid.

Since the circulatable surfaces 1, 2 are uniform as far as the extent of attraction or repulsion of water is concerned, the tack of an ink layer formed on one of those surfaces can be measured and used as indication of the water absorbing capacity of the ink. As soon as such an amount of water has been added to an ink that the ink can no longer absorb it, the tack suddenly decreases substantially. This manner of determining the water absorbing capacity proves to yield a better prediction for the water absorbing behavior in practice than known determinations of the water absorbing capacity on the basis of viscosity measurement.

As to the use of a circulatable offset printing plate for taking up ink coming from the circulatable surface, and the use of a trial printing roller for making proofs directly or indirectly starting from the ink film formed, it is further observed that these may also be advantageously used in an apparatus for measuring the tack in which no provisions are present for forming the film layer from an ink supply through which the circulatable surface passes. However, the use of the ink supply in combination with the transfer of ink to an offset printing plate is of particular advantage, because in that case, the ink film is quickly replenished to compensate for ink and water taken up and, also, any disturbances of the content of one or more components of the ink layer 30 are restored again from the ink supply 30.

As the container for the ink supply 30 is partly formed by opposite portions of two revolving surfaces 1, 2, the ink film is simultaneously formed on two revolving surfaces. This offers the advantage that simultaneously, two tests can be performed on ink films of equal composition without the tests influencing each other substantially.

The outlet gap of the container formed by the cylinders 3, 4 for retaining the ink supply, bounded by portions of the revolving surfaces 1, 2, offers the advantage that the ink film is formed in a controlled manner and that in the area of the gap, a substantial shear takes place, so that during the formation of the ink film, a final, intensive mixture of the ink takes place in the gap 36.

As the gap 36, too, is bounded on two opposite sides by two opposite portions of the revolving surfaces 1, 2, the ink films on both surfaces 1, 2 are formed in the same gap 36. Thus, differences in the manner in which the ink films are formed are eliminated as possible cause of differences between the ink films on the surfaces 1, 2.

For a controlled formation of the ink film, it is further advantageous that the portions of the revolving surfaces 1, 2 located in the area of the gap 36 move away from the ink supply 30, as a result of which the formation of the ink film takes place in the gap 36.

For obtaining identical ink films on revolving surfaces 1, 2, it is further advantageous that they move through the gap 36 at substantially identical speeds and in substantially identical directions of movement. There is further provided a transmission 43 between the cylinders 3, 4 to ensure that they move through the gap 36 at the same speed.

It is also possible to design the transmission 43 such that the revolving surfaces 1, 2 move through the gap at different speeds. Thus, an additionally strong shear action and an accordingly enhanced mixing action can be effected in the area of the gap 36, which is advantageous for some applications and in particular for a fast buildup of the film. Optionally, first, or after each change of the composition of the ink, rotation can take place at different speeds of the surfaces 1, 2 to effect a fast and even film buildup, to subsequently switch over to equal circumferential speeds to bring the films on both surfaces under equal operating conditions.

As the ink supply 30 is kept above the gap 36 and the gap 36 bounds the bottom side of the ink supply 30, it is readily guaranteed that ink is always present before the gap 36, also if the ink supply 30 is small.

It will be readily understood by anyone skilled in the art that within the framework of the invention, many alternative possibilities are conceivable. Thus, for instance, instead of circumferential surfaces of cylinders, revolving belts may be used for forming one or more circulating surfaces carrying the ink layer, and in the revolving direction or transversely thereto, these belts may or may not be flat or curved in the area where the ink supply is retained. For retaining the ink supply, it is also possible to use, for instance, a container part having an open bottom side which connects to a circulating surface and comprises a squeegee for forming a film on the circulating surface. Instead of a number of nozzles for introducing ink that are distributed over the width of the container for receiving an ink supply, there may, for instance, be provided a single nozzle, which may be mounted in a fixed position or for back and forth movement, like the head of an inkjet printer.

## Claims

1. A method for determining the behavior of a film layer of printing ink at different contents of at least one component, comprising the steps of:
applying a film layer of said printing ink to a circulating surface (1, 2),
performing tests on said film layer for determining at least one property of said printing ink,
changing the content of said at least one component in said film layer, and
performing said tests on said film layer having said changed content of said at least one component,
said film layer is formed from an ink supply (30) in contact with said circulating surface (1, 2), and
the content of said at least one component in said ink supply (30) is changed,
**characterized in that**
said ink supply (30) is taken up in a container formed by at least one wall in cooperation with said at least one circulatable surface (1, 2), at least one track portion of said circulatable surface (1, 2) circulating through said container.

2. A method according to claim 1, wherein said at least one component is added to said ink supply (30).

3. A method according to claim 2, wherein said at least one component is liquid.

4. A method according to any one of the preceding claims, wherein said at least one component is water.

5. A method according to any one of the preceding claims, wherein said tests comprise the measuring of the tack of said film.

6. A method according to any one of the preceding claims, wherein said tests comprise the steps of: transferring ink from the film layer onto a revolving offset printing plate and observing the properties of the transferred ink on said printing plate.

7. A method according to claim 6, wherein said tests comprise the steps of: transferring ink coming from the film layer onto a circulating trial printing surface (28) and making, from said trial printing surface (28), a proof on a support.

8. A method according to any one of the preceding claims, wherein said film supply is kept between facing portions of at least two circulating surfaces (1, 2).

9. A method according to any one of the preceding claims, wherein said film is formed in a gap (36) between opposite surface portions of which at least one is circulating.

10. A method according to claim 9, wherein said portions in the area of said gap (36) move away from said ink supply (30).

11. A method according to claim 10, wherein said circulating surfaces (1, 2) move through said gap (36) at substantially identical speeds.

12. A method according to claim 10, wherein said circulating surfaces (1, 2) move through said gap (36) at different speeds.

13. A method according to any one of claims 9-12, wherein said ink supply (30) is kept above said gap (36), so that the gap (36) bounds the bottom side of the ink supply (30).

14. A method according to any one of the preceding claims, wherein said at least one circulating surface (1, 2) circulates through said ink supply (30).

15. A method according to any one of the preceding claims, wherein said circulating surface (1, 2) is uniform as to the extent of retaining or repelling said at least one component.

16. An apparatus for determining the behavior of a printing ink at different contents of at least one component, comprising:
at least one circulatable surface (1, 2) for carrying a film layer of printing ink,
a set of instruments (5-8, 9-16, 25-29) for performing tests on said film layer,
**characterized by:**
at least one wall for forming, in cooperation with said at least one circulatable surface (1, 2), a container for taking up an ink supply (30) in contact with a portion of said circulatable surface (1, 2), at least one track portion of said circulatable surface (1, 2) being circulatable through said container.

17. An apparatus according to claim 16, further comprising a discharge structure (40, 41) for feeding said at least one component to said container.

18. An apparatus according to claim 16 or 17, wherein said circulatable surface (1, 2) is uniform as to the extent of attraction or repulsion of said at least one component.

19. An apparatus according to any one of claims 16-18, wherein said set of instruments (5-8) for performing tests comprises a circulatable surface (6), of which a portion is located opposite a portion of said at least one circulatable surface (1) for measuring, in cooperation with said circulatable surface (1), the tack of said film layer.

20. An apparatus according to any one of claims 16-19, further comprising a circulatable offset printing plate (16) for taking up ink coming from said circulatable surface (1, 2).

21. An apparatus according to any one of claims 16-20, wherein said set of instruments (25-29) comprises a circulatable trial printing surface (28) for taking up, in co-rotating cooperation, ink coming from said circulatable offset printing plate (16) or at least one of said circulatable surfaces (1, 2).

22. An apparatus according to claim 21, wherein said circulatable trial printing surface (28) is movable back and forth between a position in which it is supported by said circulatable offset printing plate (16) for transferring a printing pattern formed on said printing plate, and a position in which it is supported by at least one of said circulatable surfaces (1, 2).

23. An apparatus according to any one of claims 16-22, further comprising at least two of said circulating surfaces (1, 2), of which opposite portions constitute walls of said container for retaining an ink supply (30).

24. An apparatus according to any one of claims 16-23, wherein said container has an outlet gap (36), bounded on at least one side by a portion of said at least one circulating surface (1, 2).

25. An apparatus according to claim 24, wherein said gap (36) is bounded on two opposite sides by two surface portions of which said at least one is part of a circulatable surface (1, 2).

26. An apparatus according to claim 25, further comprising a drive mechanism (39) for causing said at least one circulatable surface (1, 2) to circulate in a sense such that portions in the area of said gap (36) move away from said ink supply (30).

27. An apparatus according to claims 25 or 26, including two opposite ones of said circulatable surfaces (1, 2), of which said surface portions bound said gap (36), said circulatable surfaces (1, 2) which bound said gap (36) on either side being coupled for movement in each case at speeds in a mutually fixed ratio.

28. An apparatus according to any one of claims 24-27, wherein said gap (36) forms a lower boundary of said container.

29. An apparatus according to any one of claims 24-28, wherein said gap (36) has a substantially rigidly fixed width.

## Patentansprüche

1. Verfahren zum Bestimmen des Verhaltens einer Filmschicht aus Drucktinte bei unterschiedlichen Gehalten mindestens einer Komponente, mit folgenden Schritten:
Aufbringen einer Filmschicht aus Drucktinte auf eine umlaufende Fläche (1,2),
Durchführen von Tests an der Filmschicht zum Bestimmen mindestens einer Eigenschaft der Drucktinte,
Verändern des Gehalts der mindestens einen Komponente in der Filmschicht, und
Durchführen der Tests an der Filmschicht mit dem veränderten Gehalt der mindestens einen Komponente,
wobei die Filmschicht aus einem mit der umlaufenden Fläche (1,2) in Kontakt stehenden Tintenvorrat (30) hergestellt wird, und
der Gehalt der mindestens einen Komponente in dem Tintenvorrat (30) verändert ist,
**dadurch gekennzeichnet, dass**
der Tintenvorrat (30) in einen Behälter aufgenommen ist, der aus mindestens einer Wand gebildet ist, die mit der mindestens einen drehbaren Fläche (1,2) zusammenwirkt, wobei mindestens ein Spurbereich der drehbaren Fläche (1,2) durch den Behälter umläuft.

2. Verfahren nach Anspruch 1, bei dem die mindestens eine Komponente dem Tintenvorrat (30) hinzugefügt ist.

3. Verfahren nach Anspruch 2, bei dem die mindestens eine Komponente eine Flüssigkeit ist.

4. Verfahren nach einem der vorhergehenden Ansprüche, bei dem die mindestens eine Komponente Wasser ist.

5. Verfahren nach einem der vorhergehenden Ansprüche, bei dem die Tests das Messen der Klebrigkeit des Films umfassen.

6. Verfahren nach einem der vorhergehenden Ansprüche, bei dem die Tests folgende Schritte umfassen:
Übertragen von von der Filmschicht herrührender Tinte auf eine sich drehende Offset-Druckfläche und Beobachten der Eigenschaften der auf die Druckfläche übertragenen Tinte.

7. Verfahren nach Anspruch 6, bei dem die Tests folgende Schritte umfassen:
Übertragen von Tinte von der Filmschicht auf eine sich drehende Versuchs-Druckfläche (28) und Erstellen eines Abzugs von der Versuchs-Druckfläche (28) auf einem Träger.

8. Verfahren nach einem der vorhergehenden Ansprüche, bei dem die Filmzufuhr zwischen einander zugewandten Teilen mindestens zweier umlaufender Flächen (1,2) gehalten wird.

9. Verfahren nach einem der vorhergehenden Ansprüche, bei dem der Film in einem Spalt (36) zwischen gegenüberliegenden Flächenbereichen, von denen sich mindestens einer dreht, gebildet ist.

10. Verfahren nach Anspruch 9, bei dem sich die Teile in dem Bereich des Spalts (36) von dem Tintenvorrat (30) fortbewegen.

11. Verfahren nach Anspruch 10, bei dem sich die sich drehenden Flächen (1,2) mit im wesentlichen gleichen Geschwindigkeiten durch den Spalt (36) bewegen.

12. Verfahren nach Anspruch 10, bei dem sich die sich drehenden Flächen (1,2) mit unterschiedlichen Geschwindigkeiten durch den Spalt (36) bewegen.

13. Verfahren nach einem der Ansprüche 9-12, bei dem der Tintenvorrat (30) oberhalb des Spalts (36) gehalten wird, so dass der Spalt (36) die Unterseite des Tintenvorrats begrenzt.

14. Verfahren nach einem der vorhergehenden Ansprüche, bei dem die mindestens eine sich drehende Fläche (1,2) durch den Tintenvorrat (30) zirkuliert.

15. Verfahren nach einem der vorhergehenden Ansprüche, bei dem die sich drehende Fläche (1,2) hinsichtlich des Ausmaßes des Zurückhaltens oder Abweisens der mindestens einen Komponente gleichmäßig ist.

16. Vorrichtung zum Bestimmen des Verhaltens einer Drucktinte mit unterschiedlichen Gehalten mindestens einer Komponente, mit:
mindestens einer drehbaren Fläche (1,2) zum Tragen einer Filmschicht aus Drucktinte,
ein Satz Instrumente (5-8,9-16,25-29) zum Durchführen von Tests an der Filmschicht,
**gekennzeichnet durch**
mindestens eine Wand zum Bilden, zusammen mit der mindestens einen drehbaren Fläche (1,2), eines Behälters zum Aufnehmen eines Tintenvorrats (30), die mit einem Teil der drehbaren Fläche (1,2) in Kontakt steht, wobei mindestens ein Spurbereich der drehbaren Fläche (1,2) **durch** den Behälter zirkulierbar ist.

17. Vorrichtung nach Anspruch 16, ferner mit einer Auslasseinrichtung (40,41) zum Zuführen der mindestens einen Komponente zu dem Behälter.

18. Vorrichtung nach Anspruch 16 oder 17, bei der die drehbare Fläche (1,2) hinsichtlich des Ausmaßes des Anziehens oder Abweisens der mindestens einen Komponente gleichmäßig ist.

19. Vorrichtung nach einem der Ansprüche 16-18, bei der der Instrumentensatz (5-8) zum Durchführen von Tests eine drehbare Fläche (6) aufweist, wobei ein Teil der Fläche (6) gegenüber einem Teil der mindestens einen drehbaren Fläche (1) vorgesehen ist, und zwar zum Messen der Klebrigkeit der Filmschicht in Kooperation mit der drehbaren Fläche (1).

20. Vorrichtung nach einem der Ansprüche 16-19, ferner mit einer drehbaren Offset-Druckfläche (16) zum Aufnehmen von Tinte von der drehbaren Fläche (1,2).

21. Vorrichtung nach einem der Ansprüche 16-20, bei der der Satz Instrumente (25-29) eine drehbare Versuchs-Druckfläche (28) zum Aufnehmen durch Mitdrehen von Tinte von der drehbaren Offset-Druckfläche (16) oder mindestens einer der drehbaren Flächen (1,2) aufweist.

22. Vorrichtung nach Anspruch 21, bei der die drehbare Versuchs-Druckfläche (28) zwischen einer Position, in der sie von der drehbaren Offset-Druckfläche (16) zum Übertragen eines auf der Druckfläche ausgebildeten Druckmusters gehalten wird, und einer Position, in der sie von mindestens einer der drehbaren Flächen (1,2) gehalten wird, vor- und zurückbewegbar ist.

23. Vorrichtung nach einem der Ansprüche 16-22, ferner mit mindestens zwei der umlaufenden Flächen (1,2), wobei gegenüberliegende Teile der Flächen (1,2) Wände des Behälters zum Aufnehmen eines Tintenvorrats (30) bilden.

24. Vorrichtung nach einem der Ansprüche 16-23, bei der der Behälter einen Auslassspalt (36) aufweist, der auf mindesten einer Seite von einem Teil der einen umlaufenden Fläche (1,2) begrenzt ist.

25. Vorrichtung nach Anspruch 24, bei der der Spalt (36) auf zwei gegenüberliegenden Seiten von zwei Flächenbereichen begrenzt ist, von denen der mindestens eine Teil einer drehbaren Fläche (1,2) ist.

26. Vorrichtung nach Anspruch 25, ferner mit einem Antriebsmechanismus (39), der bewirkt, dass mindestens eine drehbare Fläche (1,2) in einer solchen Richtung zirkuliert, dass sich Teile in dem Bereich des Spalts (36) von dem Tintenvorrat (30) fortbewegen.

27. Vorrichtung nach Ansprüchen 25 oder 26, mit zwei einander gegenüberliegenden drehbaren Flächen (1,2), deren die Flächenbereiche den Spalt (36) begrenzen, wobei die drehbaren Flächen (1,2), die den Spalt (36) auf beiden Seiten begrenzen, derart gekoppelt sind, dass sie sich in jedem Fall mit Geschwindigkeiten mit einem festen Verhältnis relativ zueinander bewegen.

28. Vorrichtung nach einem der Ansprüche 24-27, bei der der Spalt (36) die untere Grenze des Behälters bildet.

29. Vorrichtung nach einem der Ansprüche 24-28, bei der der Spalt (36) eine im wesentlichen starr festgelegte Breite hat.

## Revendications

1. Procédé de détermination du comportement d'une couche mince d'une encre d'impression pour différentes teneurs en au moins un ingrédient, comprenant les étapes suivantes :
l'application d'une couche mince d'encre d'impression sur une surface qui circule (1, 2),
l'exécution de tests sur la couche mince pour la détermination d'au moins une propriété de l'encre d'impression,
le changement de la teneur en au moins un ingrédient de la couche mince, et
l'exécution des tests sur la couche mince ayant la teneur modifiée en un ingrédient au moins,
la couche mince étant formée à partir d'une réserve d'encre (30) qui est au contact de la surface qui circule (1, 2), et
la teneur d'un ingrédient au moins de la réserve d'encre (30) est modifiée,
**caractérisé en ce que**
la réserve d'encre (30) est prélevée dans un récipient formé par au moins une paroi qui coopère avec au moins une surface qui peut circuler (1, 2), une partie de voie au moins de la surface qui peut circuler (1, 2) circulant dans le récipient.

2. Procédé selon la revendication 1, dans lequel l'ingrédient au moins est ajouté à la réserve d'encre (30).

3. Procédé selon la revendication 2, dans lequel l'ingrédient au moins est liquide.

4. Procédé selon l'une quelconque des revendications précédentes, dans lequel l'ingrédient au moins est l'eau.

5. Procédé selon l'une quelconque des revendications précédentes, dans lequel les tests comprennent la mesure de l'adhésivité de la couche mince.

6. Procédé selon l'une quelconque des revendications précédentes, dans lequel les tests comprennent les étapes suivantes : le report d'encre de la couche mince sur un cliché d'impression offset qui tourne, et l'observation des propriétés de l'encre reportée sur le cliché d'impression.

7. Procédé selon la revendication. 6, dans lequel les tests comprennent les étapes suivantes : le report d'encre provenant de la couche mince sur une surface d'impression d'essai (28) qui circule et l'exécution, à partir de la surface d'impression d'essai (28), d'une épreuve sur un support.

8. Procédé selon l'une quelconque des revendications précédentes, dans lequel la réserve de la couche mince est conservée entre les parties en regard d'au moins deux surfaces qui circulent (1, 2).

9. Procédé selon l'une quelconque des revendications précédentes, dans lequel la couche mince est formée dans un espace (36) délimité entre des parties de surface opposées dont l'une au moins circule.

10. Procédé selon la revendication 9, dans lequel les parties qui se trouvent dans la région dudit espace (36) s'écartent de la réserve d'encre (30).

11. Procédé selon la revendication 10, dans lequel les surfaces qui circulent (1, 2) se déplacent dans ledit espace (36) à des vitesses pratiquement identiques.

12. Procédé selon la revendication 10, dans lequel les surfaces qui circulent (1, 2) se déplacent dans ledit espace (36) à des vitesses différentes.

13. Procédé selon l'une quelconque des revendications 9 à 12, dans lequel la réserve d'encre (30) est maintenue au-dessus dudit espace (36), si bien que cet espace (36) délimite le côté inférieur de la réserve d'encre (30).

14. Procédé selon l'une quelconque des revendications précédentes, dans lequel la surface qui circule au moins (1, 2) circule dans la réserve d'encre (30).

15. Procédé selon l'une quelconque des revendications précédentes, dans lequel la surface qui circule (1, 2) présente une uniformité d'amplitude de retenue ou de répulsion dudit ingrédient au moins.

16. Appareil de détermination du comportement d'une encre d'impression à des teneurs différentes en au moins un ingrédient, comprenant :
au moins une surface qui peut circuler (1, 2), destinée à porter une couche mince d'une encre d'impression, et
un ensemble d'instruments (5-8, 9-16, 25-29) destinés à exécuter des tests sur la couche mince,
**caractérisé par** :
au moins une paroi destinée à former, en coopération avec ladite surface qui peut circuler au moins (1, 2), un récipient destiné à contenir une réserve d'encre (30) au contact d'une partie de la surface qui peut circuler (1, 2), une partie de voie au moins de la surface qui peut circuler (1, 2) pouvant circuler dans le récipient.

17. Appareil selon la revendication 16, comprenant en outre une structure d'évacuation (40, 41) destinée à transmettre ledit ingrédient au moins au récipient.

18. Appareil selon la revendication 16 ou 17, dans lequel la surface qui peut circuler (1, 2) est uniforme dans son amplitude d'attraction ou de répulsion dudit ingrédient au moins.

19. Appareil selon l'une quelconque des revendications 16 à 18, dans lequel l'ensemble d'instruments (5-8) destinés à exécuter les tests comprend une surface qui peut circuler (6) dont une partie est opposée à une partie de ladite surface au moins qui peut circuler (1) pour la mesure, en coopération avec la surface qui peut circuler (1), de l'adhésivité de la couche mince.

20. Appareil selon l'une quelconque des revendications 16 à 19, comprenant en outre un cliché d'impression offset qui peut circuler (16), destiné à prélever de l'encre provenant de la surface qui peut circuler (1, 2).

21. Appareil selon l'une quelconque des revendications 16 à 20, dans lequel l'ensemble d'instruments (25-29) comprend une surface d'impression d'essai (28) qui peut circuler pour prélever, en coopération par rotation simultanée, une encre provenant du cliché d'impression offset qui peut circuler (16) ou de l'une au moins des surfaces qui peuvent circuler (1, 2).

22. Appareil selon la revendication 21, dans lequel la surface d'impression d'essai qui peut circuler (28) est mobile alternativement entre une position dans laquelle elle est supportée par le cliché d'impression offset qui peut circuler (16) pour le report d'un motif d'impression formé sur le cliché d'impression, et une position dans laquelle elle est supportée par l'une au moins des surfaces qui peuvent circuler (1, 2).

23. Appareil selon l'une quelconque des revendications 16 à 22, comprenant en outre au moins deux surfaces qui peuvent circuler (1, 2) dont des parties opposées constituent des parois du récipient destiné à contenir une réserve d'encre (30).

24. Appareil selon l'une quelconque des revendications 16 à 23, dans lequel le récipient a un espace de sortie (36) délimité d'au moins un côté par une partie de la surface qui peut circuler au moins (1, 2).

25. Appareil selon la revendication 24, dans lequel ledit espace (36) est délimité aux deux cotés opposés par deux parties de surface dont l'une au moins fait partie d'une surface qui peut circuler (1, 2).

26. Appareil selon la revendication 25, comprenant en outre un mécanisme d'entraînement (39) destiné à provoquer la circulation de la surface au moins qui peut circuler (1, 2) dans un sens tel que les parties de la surface dudit espace (36) s'écartent de la réserve d'encre (30).

27. Appareil selon la revendication 25 ou 26, comprenant deux surfaces opposées qui peuvent circuler (1, 2) dont des parties de surface délimitent ledit espace (36), les surfaces qui peuvent circuler (1, 2) qui délimitent ledit espace (36) de part et d'autre étant couplées afin qu'elles se déplacent dans chaque cas à des vitesses présentant un rapport fixe.

28. Appareil selon l'une quelconque des revendications 24 à 27, dans lequel ledit espace (36) forme une limite inférieure du récipient.

29. Appareil selon l'une quelconque des revendications 24 à 28, dans lequel ledit espace (36) a une largeur fixée de manière pratiquement rigide.
